(19) Europäisches Patentamt
European Patent Office
Office européen des brevets

(11) Numéro de publication: **0 213 995**
**B1**

(12) # FASCICULE DE BREVET EUROPEEN

(45) Date de publication du fascicule du brevet: **06.12.89**

(51) Int. Cl.⁴: **C07D 417/14,** A61K 31/425

(21) Numéro de dépôt: **86401601.9**

(22) Date de dépôt: **17.07.86**

(54) **Nouveaux dérivés de la 3-amino 2-oxoazétidinone comportant, en position 1, un radical hétérocyclique azoté, leur procédé de préparation et leur application comme médicaments.**

(30) Priorité: **18.07.85 FR 8511000**

(43) Date de publication de la demande: **11.03.87 Bulletin 87/11**

(45) Mention de la délivrance du brevet: **06.12.89 Bulletin 89/49**

(84) Etats contractants désignés: **AT BE CH DE FR GB IT LI LU NL SE**

(56) Documents cités:
**EP-A- 0 095 778**
**EP-A- 0 114 128**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris(FR)**

(72) Inventeur: **Teutsch, Jean-Georges, Résidence Lavoisier - Bât. 3 3, rue Lavoisier, F-93500 Pantin(FR)**
Inventeur: **Klich, Michel, 9, rue Robert Jumel, F-93250 Villemomble(FR)**
Inventeur: **Chantot, Jean-François, 2, Allée Eole, F-77410 Gressy en France(FR)**

(74) Mandataire: **Bourgouin, André et al, Département des Brevets ROUSSEL UCLAF B.P. no 9, F-93230 Romainville(FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Dans le brevet n° 2 539 128 déposé en France le 10 Janvier 1983, la société demanderesse a décrit des nouveaux dérivés de la 3-amino 2-oxoazétidinone comportant, en position 1, un radical hétérocyclique azoté, leur procédé de préparation, leur application comme médicaments et les produits intermédiaires nécessaires à leur préparation.

Les produits décrits dans ce brevet répondent à la formule générale suivante :

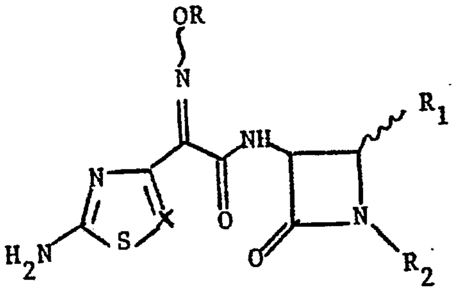

dans laquelle R représente un atome d'hydrogène, un radical alkyle, alkényle ou alkynyle ayant au plus 12 atomes de carbone, éventuellement substitué, un radical cycloalkyle ayant de 3 à 8 atomes de carbone éventuellement substitué ou un radicale acyle ou aryle ou aralkyle, éventuellement substitué,
$R_1$ représente un atome d'hydrogène, un radical alkyle, alkényle ou alkynyle ou thioalkyle ayant au plus 12 atomes de carbone, éventuellement substitué, un radical carboxy libre, estérifié ou salifié, un radical aryle, acyle ou carbamoyle, ces-radicaux étant éventuellement substitués,
$R_2$ représente un hétérocycle azoté éventuellement substitué et comportant au moins un hydrogène acide, X représente un radical CH ou un atome d'azote, les traits ondulés signifient que le radical OR peut se trouver sous la forme syn ou anti et que les produits peuvent se trouver sous la forme cis ou trans ou sous la forme d'un mélange cis-trans, ces produits étant sous forme racémique ou optiquement active, ainsi que les sels de ces produits avec les bases et les acides.

La société demanderesse a maintenant découvert qu'une classe particulière de produits compris dans la formule générale ci-dessus présente des propriétés pharmacologiques particulièrement intéressantes.

La présente demande a donc pour objet les produits répondant à la formule I :

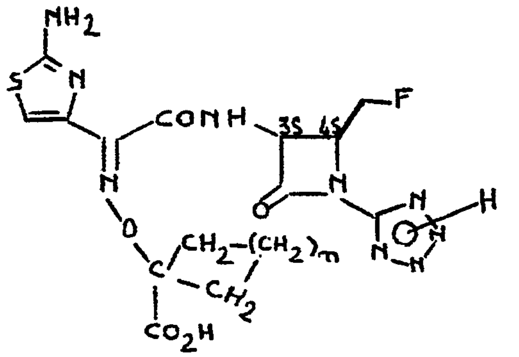

(I)

dans laquelle n représente un nombre entier de 0 à 3 ainsi que leurs sels avec les bases et les acides.

Parmi les sels des produits de formule (I) avec les bases, on peut citer plus particulièrement les sels de sodium et de potassium.

En plus des sels de sodium et de potassium, on peut également citer les sels de lithium, calcium, magnésium, ammonium.

On peut également citer les sels de base organiques telle que la triméthylamine, la diéthylamine, la triéthylamine, la méthylamine, la propylamine, la N,N-diméthyl-éthanolamine, le tris (hydroxyméthyl) aminométhane, l'éthanolamine, la pyridine, la picoline, la dicyclohexylamine, la N', N'-dibenzyléthylènediamine, la morpholine, la benzylamine, la procaïne, la lysine, l'arginine, l'histidine, la N-méthyl glucamine.

Les produits de formule I peuvent se présenter sous forme de sels d'acides organiques ou minéraux puisque ces produits contiennent un radical amino salifiable.

Parmi les acides avec lesquels on peut salifier le groupement amino des produits de formule I, on peut citer entre autres, les acides acétique, trifluoroacétique, maléïque, tartrique, méthanesulfonique, benzènesulfonique, p-toluènesulfonique, chlorhydrique, bromhydrique, sulfurique, phosphorique. Les produits de formule I peuvent également se présenter sous forme de sels internes.

Parmi les produits de formule I, on préfère particulièrement les produits suivants :

- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy/cyclopropane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/ 2-oxo éthylidène/amino/oxy/cyclobutane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy cyclopentane carboxylique et ses sels.

La présente demande a également pour objet un procédé de préparation des produits de formule I caractérisé en ce que l'on traite un produit de formule II :

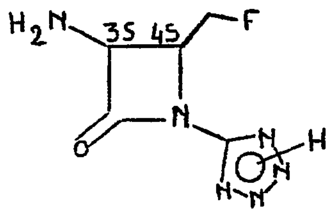

(II)

par un produit de formule III :

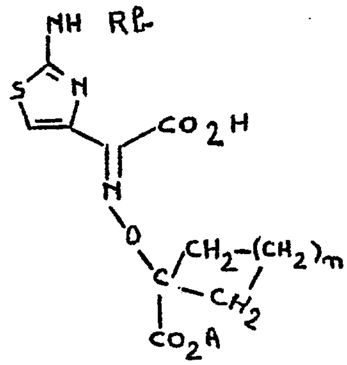

(III )

dans laquelle n est défini comme précédemment, Rb représente un atome d'hydrogène ou un groupement protecteur du radical amino et A représente un atome d'hydrogène ou un groupement ester facilement clivable, pour obtenir un produit de formule IV :

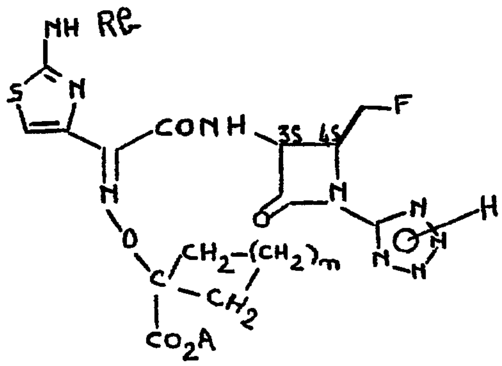

(IV )

dans laquelle n, Rb et A ont la signification précédente, produit que l'on soumet si nécessaire à l'une quelconque des réactions suivantes, dans un ordre quelconque :

a/. coupure par hydrolyse, hydrogénolyse ou par action de la thiourée du ou des groupements protecteurs que peuvent représenter Rb et A ;
b/. salification du radical carboxy ;
c/. salification par un acide du radical amino.

Le radical Rb représente, de préférence un groupement protecteur du radical amino qui peut être choisi dans la liste figurant dans le brevet français n° 2 539 128.

Cette liste n'est pas limitative, il est évident que d'autres groupements protecteurs des amines, groupements connus en particulier dans la chimie des peptides, peuvent également être utilisés.

Parmi les groupes préférés, on peut citer les groupements formyle, acétyle, éthoxycarbonyle, mésyle,

trifluoroacétyle, chloroacétyle, trityle. On préfère particulièrement le radical trityle.

Le groupement ester facilement éliminable que peut représenter A, peut être par exemple l'ester formé avec les radicaux butyle, isobutyle, tert-butyle, pentyle, hexyle, acétoxyméthyle, propionyloxyméthyle, butyryoxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, βββ-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chlorophényle, tolyle, tert-butylphényle.

Parmi les radicaux préférés, on peut citer les radicaux diphénylméthyle et tert-butyle.

Dans un mode préférentiel d'exécution du procédé, on traite le produit de formule (II) par un dérivé fonctionnel d'un produit de formule (III). Ce dérivé fonctionnel peut être par exemple un halogénure, un anhydride symétrique ou mixte, un amide ou un ester activé.

Comme exemple d'anhydride mixte, on peut citer par exemple celui formé avec le chloroformiate d'isobutyle et celui formé avec le chlorure de pivaloyle et les anhydrides mixtes carboxylique sulfonique formés par exemple avec le chlorure de tosyle. Comme exemple d'ester activé, on peut mentionner l'ester formé avec le 2,4-dinitrophényl et celui formé avec l'hydroxybenzothiazole.

Comme exemple d'halogénure, on peut citer le chlorure ou le bromure.

On peut citer également l'azide d'acide ou l'amide d'acide.

L''anhydride peut être formé in situ par action de carbodiimide NN'-disubstitué, par exemple la N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'qcide halohydrique est libérée au cours de la réaction, on réalise la réaction de préférence en présence d'une base telle que la soude, la potasse, les carbonate et carbonate acides de sodium ou de potassium, l'acétate de sodium, la triéthylamine, la pyridine, la morpholine ou la N-méthylmorpholine.

La température de réaction est en général inférieure ou égale à la température ambiante.

On utilise de préférence un anhydride mixte carboxylique sulfonique.

Selon les valeurs de Rb et de A, les produits de formule (IV) peuvent ou non constituer des produits de formule (I).

Les produits de formule (IV) constituent des produits de formule (I) lorsque Rb et A représentent un atome d'hydrogène.

Dans les autres cas, l'action sur le produit de formule (IV) d'un ou plusieurs agents d'hydrolyse, d'hydrogénolyse ou de la thiourée a pour but d'éliminer le radical Rb lorsque celui-ci représente un radical protecteur du radical amino et d'éliminer le radical A lorsque celui-ci représente un groupement ester.

La nature des réactifs à mettre en jeu dans tous ces cas est bien connue de l'homme de métier et est illustrée dans la partie expérimentale.

On donne ci-après une énumération non exhaustive des moyens pouvant être mis en oeuvre pour éliminer les différents groupements.

L'élimination du groupe Rb peut être effectuée par hydrolyse, celle-ci étant acide, basique ou utilisant l'hydrazine.

On utilise préférentiellement l'hydrolyse acide pour éliminer les groupements alkyle, alkoxy, aralkyle et cycloalkoxycarbonyle, éventuellement substitués, tels que tert-pentyloxycarbonyle ou tert-butyloxycarbonyle, les groupements aralcoxycarbonyle éventuellement substitués tels que benzyloxycarbonyle, les groupements trityle, diphénylméthyle, tert-butyle ou 4-méthoxybenzyle.

L'acide que l'on utilise peut être choisi dans le groupe constitué par les acides chlorhydrique, benzènesulfonique ou paratoluène sulfonique, formique ou trifluoroacétique. On peut cependant utiliser d'autres acides minéraux ou organiques. On utilise de préférence l'acide trifluoroacétique ou l'acide formique.

L'hydrolyse basique est utilisée préférentiellement pour éliminer les groupements acyles tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Le groupement Rb peut également être éliminé par le système zincacide acétique (pour le groupement trichloroéthyle), les groupements diphénylméthyle, benzyloxycarbonyle sont éliminés de préférence par l'hydrogène en présence d'un catalyseur.

Le groupement chloroacétyle est éliminé par action de la thiourée en milieu neutre ou acide selon le type de réaction décrit par MASAKI J.A.C.S., 90, 4508 (1968).

On peut également utiliser d'autres méthodes de déprotection connues dans la littérature.

L'élimination du radical A lorsque celle-ci est nécessaire, est réalisée dans des conditions semblables à celles décrites précédemment pour l'élimination de Rb.

On peut utiliser, entre autres, l'hydrolyse acide pour éliminer les radicaux alkyle ou aralkyle éventuellement substitués.

On utilise préférentiellement un acide choisi dans le groupe formé par les acides chlorhydrique, formique, trifluoroacétique et paratoluène sulfonique.

Les autres valeurs des radicaux Rb ou A sont, lorsque cela est désiré, éliminées selon les procédés connus de l'homme de métier. On opère de préférence dans des conditions modérées, c'est-à-dire, à température ambiante ou en chauffant légèrement.

Naturellement, on peut lorsque par exemple Rb ou A sont des groupements éliminables appartenant à des types différents, faire agir sur les produits (IV) plusieurs agents envisagés dans les énumérations précédentes.

La salification éventuelle des produits de formule (I), par les acides ou les bases, peut être effectuée selon les méthodes usuelles, décrites par exemple dans le brevet français 2 539 128.

La salification peut, par exemple, être obtenue par action sur un produit sous forme acide ou sur un solvat, par exemple le solvat éthanolique, ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phsophate trisodique, On peut également faire appel à des sels d'acides organiques.

On trouvera une liste de tels sels d'acides organiques par exemple dans le brevet français 2 476 087.

On utilise de préférence comme sels de sodium, l'acétate de sodium, le 2-éthyl héxanoate de sodium ou de diéthylacétate de sodium.

La salification peut également être obtenue par action d'une base organique ou d'un acide aminé.

Les produits de formule générale (I) possèdent une très bonne activité antibiotique sur les bactéries gram (-), notamment sur les bactéries coliformes, les klebsiella, les salmonella et proteus.

Ces produits peuvent notamment être utilisés comme médicaments dans le traitement des colibacilloses et infections associées, dans les infections à proteus, à klebsiella et à salmonella et dans d'autres affections provoquées par des bactéries à gram (-).

La présente invention a donc égélement pour objet, à titre de médicaments et notamment de médicaments antibiotiques, les produits de formule (I), tels que définis ci-dessus, ainsi que leurs sels pharmaceutiquement acceptables.

L'invention a particulièrement pour objet, à titre de médicaments et notamment de médicaments antibiotiques,

- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy/ cyclopropane carboxylique et ses sels pharmaceutiquement acceptables,
- l'acide (3S,4S) 1-///1-(2-amino 4-thiazolyl) 2-//(4-fluorométhyl 2-oxo 1-tétrazolyl 3-azétidinyl)/amino/ 2-oxo éthylidène/amino/oxy/cyclobutane carboxylique et ses sels pharmaceutiquement acceptables,
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy cyclopentane carboxylique et ses sels pharmaceutiquement acceptables.

L'invention s'étend aux compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses.

Elles peuvent être solides ou liquides et se présenter sous les formes pharmaceutiques couramment utilisées en médecine humaine comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous la forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 2, ou encore comprise entre 0,500 g et 1 g trois fois par jour, par voie intramusculaire.

Les produits de formule (I) et leurs sels peuvent également être utilisés comme désinfectants des instruments chirurgicaux.

Les produits de formule III sont décrits notamment dans le brevet français 2.445.835.

Les produits de formule II, peuvent être préparés à partir de la (3S,4S) 3-amino 4-fluorométhyl 2-azétidinone dont la préparation est décrite dans le brevet français 2 558 467. Le procédé de préparation des produits de formule II, à partir de ce produit, est décrit dans la demande européenne EP 0114.128.

Les exemples suivants illustrent l'invention, sans toutefois la limiter.

Exemple 1 : (3S,4S) acide 1-///1-(2-amino 4-thiazolyl) 2-//4-fluorométhyl 2-oxo 1-tétrazolyl 3-azétidinyl/amino/ 2-oxoéthylidène/amino/ oxy/ cyclopropane carboxylique

Stade A : (3S,4S) 1/// 1-(2-tritylamino 4-thiazolyl) 2-//(4-fluorométhyl 2-oxo 1-tétrazolyl 3-azétidinyl)/amino/2-oxoéthylidène amino/ oxy/ cyclopropane carboxylate de 1,1-diméthyléthyle.

On agite jusqu'à solubilisation un mélange de 1,96 g d'acide 2-(1-tert-butoxycarbonyl cycloprop -1-oxyimino)-2-(2-tritylaminothiazol 4-yl) acétique isomère syn décrit dans le brevet français 2.445.835 préparation 7, 30 cm3 d'acétone anhydre et 0,5 cm3 de triéthylamine. On ajoute ensuite 0,765 g de chlorure de tosyle puis agite 10 minutes à température ambiante. On ajoute enfin 0,93 g de 3-amino 4-fluorométhyl 1-(1H-tétrazol-5-yl) 2-azétidinone 3S, 4S sous forme de chlorhydrate en solution dans 15 cm3 d'acétonitrile contenant 1,5 cm3 de triéthylamine.

Après 5 minutes d'agitation à température ambiante, on ajoute 0,4 cm3 d'acide acétique pur. On concentre presque à sec, reprend par 100 cm3 d'acétate d'éthyle. On lave la phase organique à l'eau, sèche et évapore à sec. On obtient 2 g d'une huile que l'on purifie par chromatographie sur silice éluant : chlorure de méthylène-méthanol 95:5 à 0,5 % d'acide acétique. On obtient 1,6 g de résine que l'on utilise comme telle pour le stade suivant :

Stade B : (3S,4S) acide 1-///1-(2-amino 4-thiazolyl) 2-//4-fluorométhyl 2-oxo 1-tétrazolyl 3-azétidinyl/amino/ 2-oxoéthylidène/amino/ oxy/ cyclopropane carboxylique.

On agite 15 minutes à température ambiante un mélange de 1,6 g de produit obtenu au stade A et 8 cm3 d'acide trifluoroacétique. On évapore à sec et reprend le mélange réactionnel par 2 fois 20 cm3 d'acétonitrile, afin éliminer l'acide trifluoroacétique. On ajoute 15 cm3 d'acide formique à 66 %, agite 30 minutes à 40°C, laisse revenir à température ambiante, filtre le triphénylcarbinol et concentre le filtrat au quart de son volume. On chromatographie sur une colonne de résine. On élue à l'eau, puis avec un mélange eau-méthanol (8:2). On obtient 0,32 g de produit sous forme de résine.
Spectre RMN 90 $MH_z$ dans le DMSO.

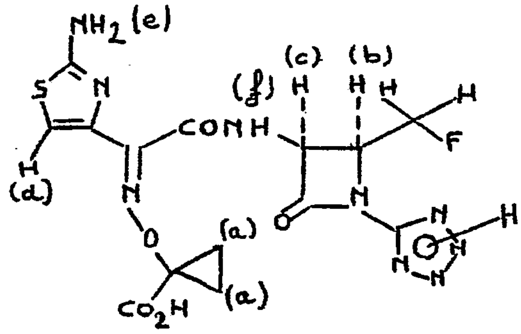

(a) 1,33 ppm
(b) 4,44 à 5,11 ppm
(c) 5,62 à 5,76 ppm
(d) 6,83 ppm
(e) 7,29 ppm
(f) 9,32 ppm (d J = 9 $H_z$)

Le chlorhydrate de 3-amino 4-fluorométhyl 1-(1H tétrazol 5-yl) 2-azétidinone 3S, 4S employé au départ du stade A a été préparé comme indiqué à l'exemple 11 de la demande de brevet européen 0 114 128 à partir de la 3-amino 4-fluorométhyl 2-azétidinone (3S,4S). Cette azétidinone a été préparée comme dans le brevet français 2 558 467 (sous forme de chlorhydrate).

Exemple 2 :(3S,4S) acide 1-///1-(2-amino 4-thiazolyl) 2-//(4-fluorométhyl 2-oxo 1-tétrazolyl 3-azétidinyl)/amino/ 2-oxoéthylidène/ amino// oxy/ cyclobutane carboxylique.

Stade A : (3S,4S) 1-///1-(2-tritylamino 4-thiazolyl) 2-//(4-fluorométhyl 2-oxo 1-tétrazolyl 3-azétidinyl)/

amino/ 2-oxoéthylidène/ amino/ oxy/ cyclobutane carboxylate de 1,1-diméthyléthyle.

a/. On maintient 25 minutes à 50°C un mélange de 2,6 g de (3S,4S) 1-(1-benzyl tétrazol 5-yl) 3-tritylamino 4-fluorométhyl 2-azétidinone, 50 cm3 d'acétonitrile et 4,9 cm3 d'acide chlorhydrique N. On distille à sec sous vide, reprend à l'acétonitrile, puis distille à nouveau. On obtient un résidu blanc cristallisé que l'on empâte à l'éther. On essore et redissout dans 40 cm3 de méthanol (solution A).

b/. On introduit 400 mg de palladium à 18% sur charbon actif dans 10 cm3 de méthanol, puis ajoute la solution A. On chauffe à 40°C et agite sous barbotage d'hydrogène pendant 30 minutes. On filtre de catalyseur et distille à sec sous pression réduite à 40°C. On reprend le résidu à l'acétonitrile et distille à nouveau, puis sèche sous pression réduite.

c/. On mélange 2,8 g d'acide 2-(1-tert-butoxy carbonyl cyclobut 1-oxyimino) 2-(2-tritylaminothiazol 4-yl acétique-isomère syn décrit dans le brevet français 2.445.835, préparation 6, 56 cm3 d'acétone et 0,7 cm3 de triéthylamine.
On ajoute après dissolution 0,92 g de chlorure de tosyle, puis agite 25 minutes à température ambiante (solution C).

d/. On mélange le produit obtenu ci-dessus en b/ avec 35 cm3 d'acétonitrile et 2 cm3 de triéthylamine, puis on ajoute en une fois la solution C obtenue ci-dessus. On agite 2 heures à température ambiante, filtre l'insoluble, puis distille à sec sur pression réduite.

On chromatographie le produit obtenu sur silice, éluant : chlorure de méthylène-méthanol 95:5 à 0,5% d'acide acétique et obtient 3 g de produit attendu.

Stade B : (3S,4S) acide 1-///1-(2-amino 4-thiazolyl) 2-//(4 fluorométhyl 2-oxo 1-tétrazolyl 3-azétidinyl)/ amino/ 2-oxoéthylidène/ amino/ oxy/ cyclobutane carboxylique.

On agite 10 minutes à température ambiante un mélange de 0,78 g de produit obtenu au stade A dans 2,5 g d'acide trifluoroacétique, puis distille rapidement à 30°C sous pression réduite.

On reprend par 5,5 cm3 d'acide formique à 66%, chauffe à 50°C et agite pendant 15 minutes, puis laisse revenir à température ambiante en 30 minutes.

On essore l'insoluble et distille à sec à 30°C sous pression réduite. On reprend à l'éthanol, concentre à sec et obtient 0,5 g de produit brut que l'on met en solution dans le minimum d'acide formique dilué et filtre sur résine en éluant à l'eau, puis successivement avec une solution aqueuse à 10 %, 20 %, 30 %, 40 % et 50 % de méthanol.

On concentre à sec, obtient 0,29 g de produit que l'on empâte dans l'éther, essore après 15 minutes et sèche sous pression réduite.

On obtient 0,286 g deproduit attendu F 220°C.

| | | | | | | |
|---|---|---|---|---|---|---|
| Analyse | $C_{15}H_{16}N_9O_5FS$ = 453,41 | | | | | |
| Calculé | C% | 39,73 | H% | 3,56 | S% | 7,07 |
| Trouvé | | 39,8 | | 3,6 | | 7,1 |

/ α / D = -33,5° + 2° (c = 0,5 % dans le méthanol).

Le (3S,4S) 1-(1-benzyltétrazol 5-yl) 3-tritylamino 4-fluorométhyl 2-azétidinone utilisé au départ du stade A a été préparé comme indiqué à l'exemple 11 de la demande de brevet européen EP 0.114.128 à partir de la (3S,4S) 3-amino 4-fluorométhy 2-azétidinone dont la préparation est donnée dans le brevet français 2 558 467.

Le (3S,4S) 1-(1-benzyltétrazol 5-yl) 3-tritylamino 4-fluorométhyl 2-azétidinone présente les caractéristiques physicochimiques suivantes :
F = 146°C.
/ α / $^{20}$D = -41,5⌑ ± 2⌑ (c = 0,5 % dans $CHCL_3$).

Exemple 3 : (3S,4S) acide 1-//1-(2-amino 4-thiazolyl) 2-//4- fluorométhyl 2-oxo 1-tétrazolyl 3-azétidinyl)/ amino/ 2-oxo éthylidène/ amino/ oxy/ cyclopentane carboxylique.

Stade A : (3S,4S) 1-///1-(2-tritylamino 4-thiazolyl) 2-///(4-fluorométhyl 2-oxo 1-tétrazolyl 3-azétidinyl)/ amino/ 2-oxoéthylidène/ amino/ oxy/ cyclopentane carboxylate de 1,1-diméthyléthyle.

On procède comme au stade A de l'exemple 2 à partir de 1,04 g de (3S,4S) 1-(1-benzyl tétrazol 5-yl) 3-tritylamino 4-fluorométhyl 2-azétidinone et de 1,2 g d'acide 2-(1-tert-butoxycarbonyl cyclopent 1-oxyimino) 2-(2-tritylamino thiazol 4-yl) acétique isomère syn décrit dans le brevet français 2.445.835, préparation 9.

On obtient 0,92 g de produit attendu.

Stade B : (3S,4S) acide 1-//1-(2-amino 4-thiazolyl) 2-//4-fluorométhyl 2-oxo 1-tétrazolyl 3-azétidinyl)/ amino/ 2-oxoéthylidène/ amino/ oxy/ cyclopentane carboxylique.

On procède comme au stade B de l'exemple 2 à partir de 0,9 g de produit obtenu au stade A ci-dessus. On obtient finalement 0,308 g de produit attendu F ≈ 220°C.
Spectre RMN : 90 MHz dans le DMSO.

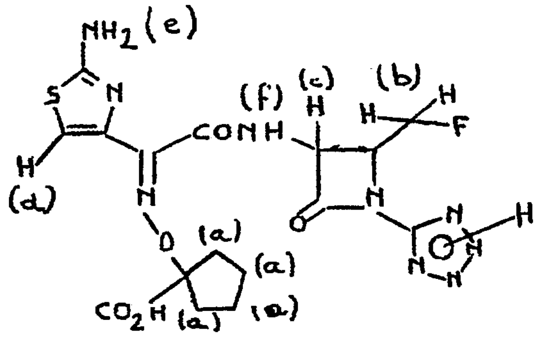

| | | | | | |
|---|---|---|---|---|---|
| (a) | entre 1,7 | et | 2,03 | ppm | |
| (b) | 4,62 | à | 5,13 | ppm | |
| (c) | 5,57 | à | 5,73 | ppm | |
| (d) | 6,75 | ppm | | | |
| (e) | 7,3 | ppm | | | |
| (f) | 9,48 | ppm | (d J = 9 Hz). | | |

Exemple 4 : On a réalisé une préparation pour injection de formule :

| | |
|---|---|
| – (3S, 4S) acide 1-///1-(2-amino 4-thiazolyl) 2-// (4-fluorométhyl 2-oxo 1-tétrazolyl 3-azétidinyl)/ amino/2-oxo éthylidène/amino/oxy/ cyclobutane carboxylique | 500 mg |
| – excipient aqueux | 5 $cm^3$ |

Exemple 5 : On a réalisé des gélules répondant à la formule :

| | |
|---|---|
| – (3S, 4S) acide 1-///1-(2-amino 4-thiazolyl) 2-// (4-fluorométhyl 2-oxo 1-tétrazolyl 3-azétidinyl)/ amino/2-oxo éthylidène/amino/oxy/ cyclobutane carboxylique | 250 mg |
| – excipient q.s.p. une gélule terminée | 400 mg |

ETUDE PHARMACOLOGIQUE DES PRODUITS DE L'INVENTION.

Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarante-huit heures dans une l'étuve à 37°C, l'inhibition de la croissance est appréciée par transillumination, ce qui permet de déterminer les concentrations minimales inhibitrices (C. M. I.) exprimées en g/cm3.

Les résultats suivants sont obtenus :

| Souches | CMI en g/cm³ | |
|---|---|---|
| | Produit de l'ex. 1 | Produit de l'ex. 2 |
| Pse. aeruginosa 1771 | 5 | 2,5 |
| Pse. aeruginosa 1771m | 0,15 | 0,15 |
| Pse. aeruginosa 9027 | 20 | 10 |
| E. coli 1894 | 0,08 | 0,04 |
| E. coli 078 | ≤ 0,04 | 0,15 |
| E. coli TEM | 0,15 | 0,15 |
| E. coli 1507 E | 0,08 | 0,15 |
| E. coli DC 0 | 0,15 | 0,15 |
| E. coli DC 2 | 0,08 | 0,08 |
| Salm. thyphimurimum MZ 11 | 0,15 | 0,3 |
| Kle. pneumoniae 52145 | 0,08 | 0,15 |
| Kle. aerogenes 1082 E | 0,15 | 0,3 |
| Kle. aerogenes 1522 E | 0,3 | 0,3 |
| Ent. cloacae P99 | 2,5 | 5 |
| Ent. cloacae 1321 E | 0,08 | 0,15 |
| Serratia RG 2532 | 0,15 | 0,15 |
| Pro. nurabilis A 235 | ≤ 0,04 | ≤ 0,02 |
| Pro. vulgaris A 232 | ≤ 0,04 | ≤ 0,02 |
| Porvidencia DU 48 | 0,08 | 0,15 |

Pse = Pseudomonas; E = Escherichia; Salm = Salmonella; Kle = Klebsiella; Ent = Enterobacter; Pro = Proteus.

**Revendications pour les Etats contractants: BE, CH, DE, FR, GB, IT, LI, LU, NL, SE**

1 -Les produits 3S, 4S répondant à la formule I :

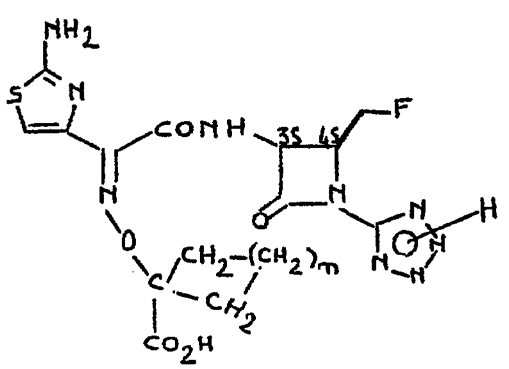

(I )

dans laquelle n représente un nombre entier de 0 à 3 ainsi que leurs sels avec les bases et les acides.

2 - L'un quelconque des produits de formule I, dont les noms suivent :
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy/cyclopropane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/ 2-oxo éthylidène/amino/oxy/cyclobutane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxycyclopentane carboxylique et ses sels.

3 - Procédé de préparation des produits de formule I telle que définie à la revendication 1 caractérisé en ce que l'on traite un produit de formule II :

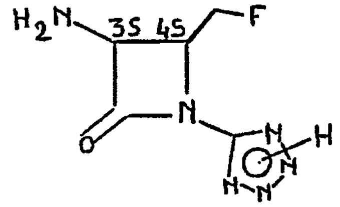
(II)

par un produit de formule III :

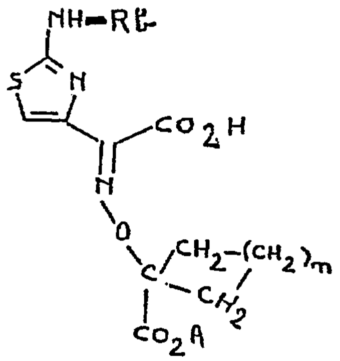
(III )

dans laquelle n est défini comme précédemment, Rb représente un atome d'hydrogène ou un groupement protecteur du radical amino et A représente un atome d'hydrogène ou un groupement ester facilement clivable pour obtenir un produit de formule IV :

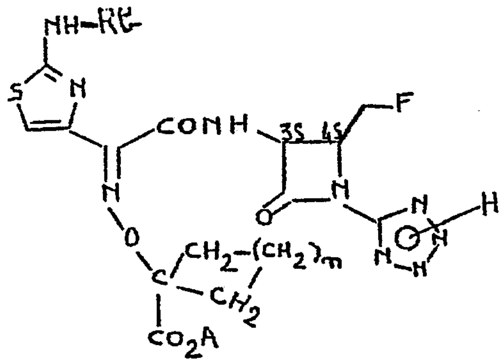
(IV )

dans laquelle n, Rb et A ont la signification précédente, produit que l'on soumet si nécessaire à l'une quelconque des réactions suivantes, dans un ordre quelconque :
a/. coupure par hydrolyse, hydrogénolyse ou par action de la thiourée du ou des groupements protecteurs que peuvent représenter Rb et A.
b/. salification du radical carboxy,
c/. salification par un acide du radical amino.

4 - A titre de médicaments, les produits répondant à la formule I telle que définie à la revendication 1 et leurs sels pharmaceutiquement acceptables.

5 - A titre de médicaments, les produits définis à la revendication 2 et leurs sels pharmaceutiquement acceptables.

6 - Compositions pharmaceutiques contenant, à titre de principe actif, au moins un médicament selon l'une des revendications 4 ou 5.

**Revendications pour l'Etat contractant: AT**

1 - Procédé pour préparer les produits 3S, 4S répondant à la formule I :

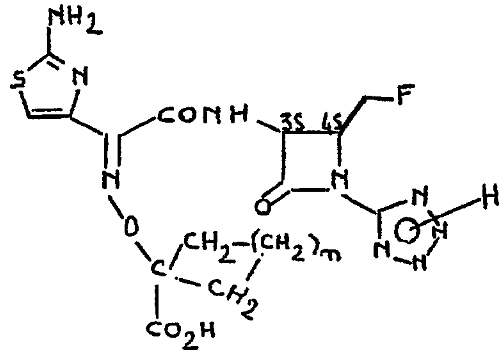

(I)

dans laquelle n représente un nombre entier de 0 à 3 ainsi que leurs sels avec les bases et les acides, caractérisé en ce que l'on traite un produit de formule II :

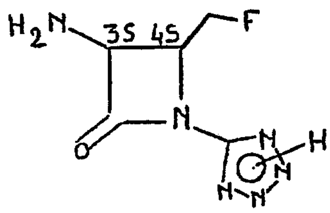

(II)

par un produit de formule III :

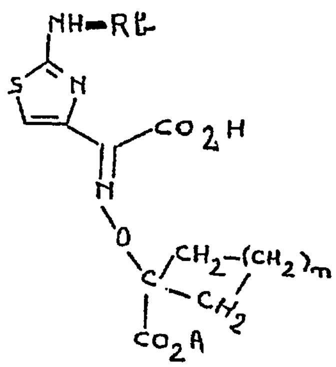

(III)

dans laquelle n est défini comme précédemment, Rb représente un atome d'hydrogène ou un groupement protecteur du radical amino et A représente un atome d'hydrogène ou un groupement ester facilement clivable pour obtenir un produit de formule IV :

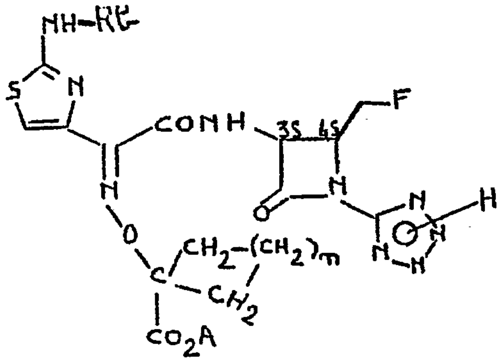

(IV)

dans laquelle n, Rb et A ont la signification précédente, produit que l'on soumet si nécessaire à l'une quelconque des réactions suivantes, dans un ordre quelconque :

a/. coupure par hydrolyse, hydrogénolyse ou par action de la thiourée du ou des groupements protecteurs que peuvent représenter Rb et A.
b/. salification du radical carboxy,
c/. salification par un acide du radical amino.

2 - Procédé selon la revendication 1, caractérisé en ce que l'on utilise au départ un produit de formule III dans laquelle Rb représente un radical trityle.

3 - Procédé selon la revendication 1 ou 2, caractérisé en ce que l'on utilise au départ un dérivé réactif du produit de formule III qui est un anhydride mixte carboxylique-sulfonique.

4 - Procédé selon l'une quelconque des revendications 1 à 3, caractérisé en ce que l'on utilise au départ des composés de formule II et III choisis de manière telle que l'on prépare les produits de formule I, dont les noms suivent :

- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy/cyclopropane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy/cyclobutane carboxylique et ses sels,
- l'acide (3S,4S) 1-///1-(2-amino 4-thiazolyl) 2-//(4-fluorométhyl 2-oxo 1-tétrazolyl 3-azétidinyl)/amino/2-oxo éthylidène/amino/oxy cyclopentane carboxylique et ses sels.

**Patentansprüche für die Vertragsstaaten: BE, CH, DE, FR, GB, IT, LI, LU, NL, SE**

1. (3S,4S)-Produkte der Formel (I)

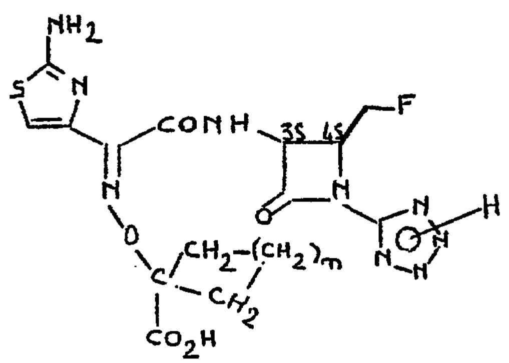

(I)

in der n eine ganze Zahl von 0 bis 3 ist, sowie ihre Salze mit Basen und Säuren.

2. Eines der Produkte der Formel (I) mit folgenden Namen:

– (3S,4S)-1-{[[1-(2-Amino-4-thiazolyl)-2-[[(4-fluormethyl-2-oxo-1-tetrazolyl-3-azetidinyl)]-amino]-2-oxoethyliden]-amino]-oxy}-cyclopropancarbonsäure und ihre Salze,
– (3S,4S)-1-{[[1-(2-Amino-4-thiazolyl)-2-[[(4-fluormethyl-2-oxo-1-tetrazolyl-3-azetidinyl)]-amino]-2-oxoethyliden]-amino]-oxy}-cyclobutancarbonsäure und ihre Salze,
– (3S,4S)-1-{[[1-(2-Amino-4-thiazolyl)-2-[[(4-fluor-methyl-2-oxo-1-tetrazolyl-3-azetidinyl)]-amino]-2-oxoethyliden]-amino]-oxy}-cyclopentancarbonsäure und ihre Salze.

3. Verfahren zur Herstellung der Produkte der Formel (I) gemäß Anspruch 1, gekennzeichnet durch Umsetzung eines Produkts der Formel (II)

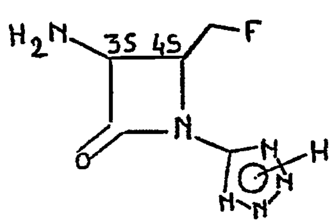

(II)

mit einem Produkt der Formel (III)

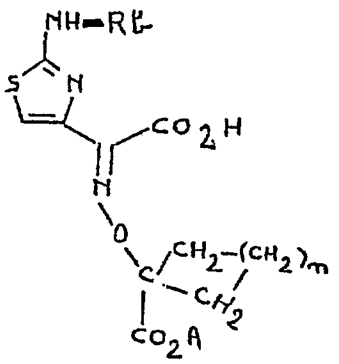

(III)

in der n wie oben definiert ist, Rb ein Wasserstoffatom oder eine Aminoschutzgruppe ist und A ein Wasserstoffatom oder eine leicht abspaltbare Estergruppe bedeutet, zu einem Produkt der Formel (IV)

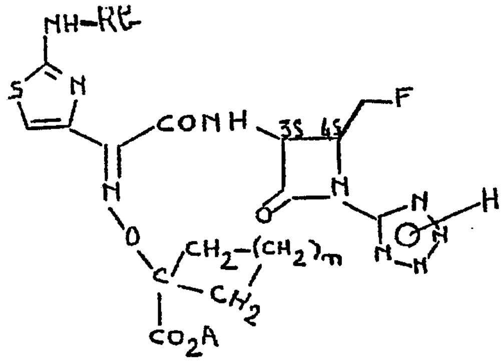

(IV)

in der n, Rb und A die oben angegebene Bedeutung haben,
ggf. Behandlung des Produkts in einer der folgenden Reaktionen in beliebiger Reihenfolge:

a) Entfernung der Schutzgruppen Rb und A durch Hydrolyse, Hydrogenolyse oder Einwirkung von Thioharnstoff,
b) Salzbildung der Carboxylgruppe,
c) Salzbildung der Aminogruppe mit einer Säure.

4. Produkte der Formel (I) gemäß Anspruch 1 und ihre pharmazeutisch verträglichen Salze als Medikamente.

5. Produkte gemäß Anspruch 2 und ihre pharmazeutisch verträglichen Salze als Medikamente.

6. Pharmazeutische Zusammensetzungen, gekennzeichnet durch mindestens ein Medikament nach Anspruch 4 oder 5 als Wirkstoff.

**Patentansprüche für den Vertragsstaat: AT**

1. Verfahren zur Herstellung der (3S,4S)-Produkte der Formel (I)

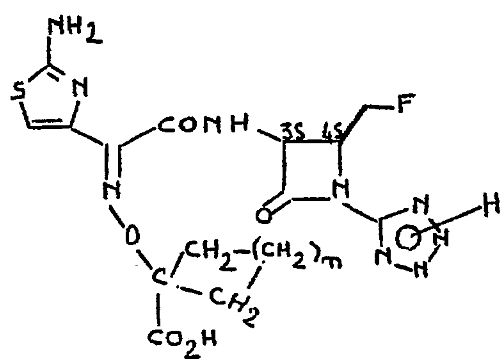

(I)

in der n eine ganze Zahl von 0 bis 3 ist, und ihrer Salze mit Basen und Säuren, gekennzeichnet durch Umsetzung eines Produkts der Formel (II)

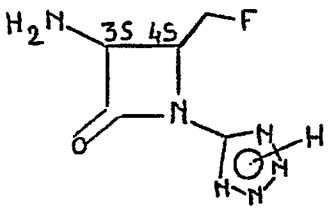

(II)

mit einem Produkt der Formel (III)

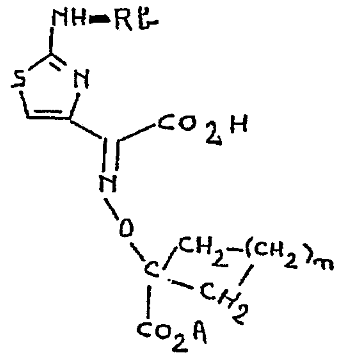

(III)

in der n wie oben definiert ist, Rb ein Wasserstoffatom oder eine Aminoschutzgruppe ist und A ein Wasserstoffatom oder eine leicht abspaltbare Estergruppe bedeutet, zu einem Produkt der Formel (IV),

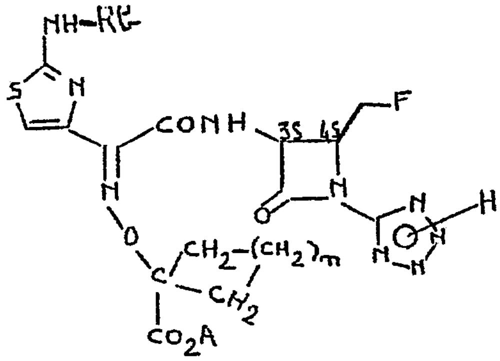

(IV)

in der n, Rb und A die oben angegebene Bedeutung haben,
ggf. Behandlung des Produkts in einer der folgenden Reaktionen in beliebiger Reihenfolge:

a) Entfernung der Schutzgruppen Rb und A durch Hydrolyse, Hydrogenolyse oder Einwirkung von Thioharnstoff,

b) Salzbildung der Carboxylgruppe,

c) Salzbildung der Aminogruppe mit einer Säure.

2. Verfahren nach Anspruch 1, gekennzeichnet durch Verwendung eines Produkts der Formel (III), in der Rb ein Tritylrest ist, als Ausgangsprodukt.

3. Verfahren nach Anspruch 1 oder 2, gekennzeichnet durch Verwendung eines reaktiven Derivats des Produkts der Formel (III), das ein gemischtes Carbonsäure-Sulfonsäure-Anhydrid ist, als Ausgangsprodukt.

4. Verfahren nach einem der Ansprüche 1 bis 3, gekennzeichnet durch Verwendung von Verbindungen der Formeln (II) und (III), die so gewählt sind, daß folgende Produkte der Formel (I) hergestellt werden:

- (3S,4S)-1-{[[1-(2-Amino-4-thiazolyl)-2-[[(4-fluormethyl-2-oxo-1-tetrazolyl-3-azetidinyl)]-amino]-2-oxoethyliden]-amino]-oxy}-cyclopropancarbonsäure und ihre Salze,
- (3S,4S)-1-{[[1-(2-Amino-4-thiazolyl)-2-[[(4-fluormethyl-2-oxo-1-tetrazolyl-3-azetidinyl)]-amino]-2-oxoethyliden]-amino]-oxy}-cyclobutancarbonsäure und ihre Salze,
- (3S,4S)-1-{[[1-(2-Amino-4-thiazolyl)-2-[[(4-fluormethyl-2-oxo-1-tetrazolyl-3-azetidinyl)]-amino]-2-oxoethyliden]-amino]-oxy}-cyclopentancarbonsäure und ihre Salze.

**Claims for the contracting States: BE, CH, DE, FR, GB, IT, LI, LU, NL, SE**

1. The 3S,4S products corresponding to the formula I:

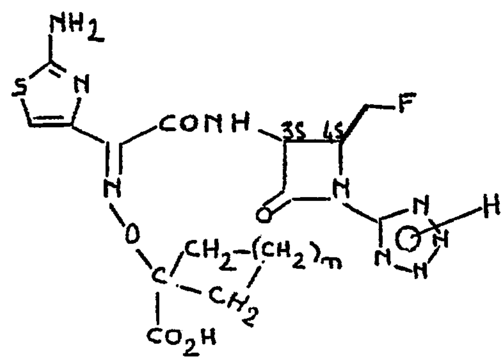

(I)

in which n represents an integer from 0 to 3, as well as their salts with bases and acids.

2. Any one of the products of formula I, of which the names follow:

– (3S,4S) 1–[[[1-(2-amino-4-thiazolyl)-2-[[(4-fluoromethyl-2-oxo-1-tetrazolyl-3-azetidinyl)]amino]-2-oxoethylidene]amino]oxy]cyclopropane carboxylic acid and its salts,

– (3S,4S) 1-[[[1-(2-amino-4-thiazolyl)-2-[[(4-fluoromethyl-2-oxo-1-tetrazolyl-3-azetidinyl)]amino]-2-oxoethylidene]amino]oxy]cyclobutane carboxylic acid and its salts,

– (3S,4S) 1-[[[1-(2-amino-4-thiazolyl)-2-[[(4-fluoromethyl-2-oxo-1-tetrazolyl-3-azetidinyl)]amino]-2-oxoethylidene]amino]oxy]cyclopentane carboxylic acid and its salts.

3. Process for the preparation of products of formula I as defined in claim 1, characterized in that a product of formula II:

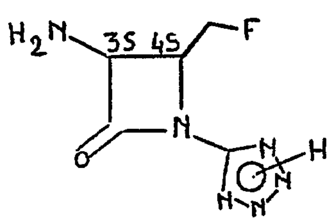

(II)

is treated with a product of formula III:

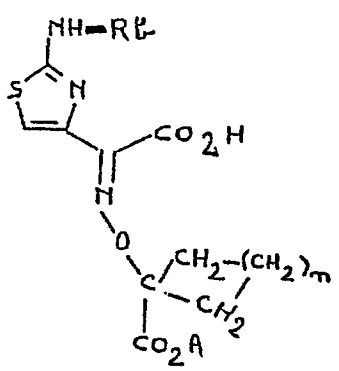

(III)

in which n is defined as previously, Rb represents a hydrogen atom or a protector group of the amino radical and A represents a hydrogen atom or an easily cleavable ester group, so as to obtain a product of formula IV:

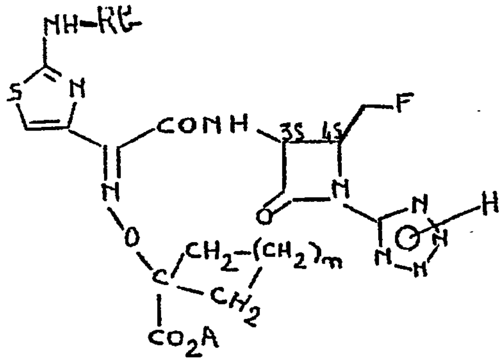

(IV)

in which n, Rb and A have the previous significance, which product is submitted, if necessary, to any of the following reactions, in any order:

a) cutting off by hydrolysis, hydrogenolysis or by the action of thiourea of the protector group or groups which Rb and A can represent;

b) salification of the carboxy radical;

c) salification by an acid of the amino radial.

4. As medicaments, the products corresponding to formula I as defined in claim 1 and their pharmaceutically acceptable salts.

5. As medicaments, the products defined in claim 2 and their pharmaceutically acceptable salts.

6. Pharmaceutical compositions containing, as active principle, at least one medicament according to one of claims 4 or 5.

**Claims for the contracting State: AT**

1. Process for preparing the 3S,4S products corresponding to formula I:

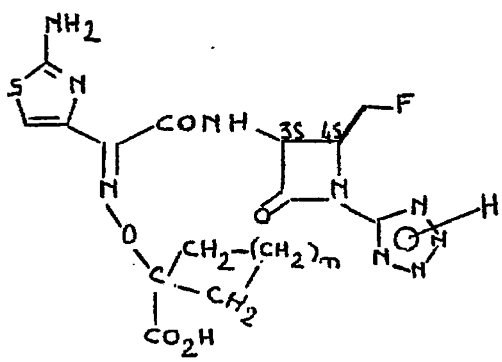

(I)

in which n represents an integer from 0 to 3, as well as their salts with bases and acids, characterized in that a product of formula II:

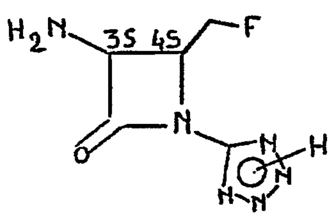

(II)

is treated with a product of formula III:

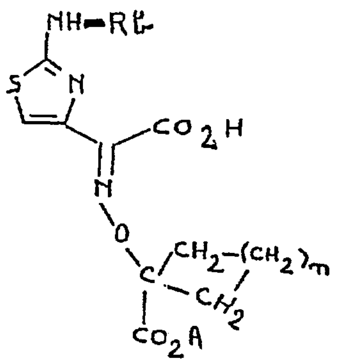

(III)

in which n is defined as previously, Rb represents a hydrogen atom or a protector group of the amino radical and A represents a hydrogen atom or an easily cleavable ester group, so as to obtain a product of formula IV:

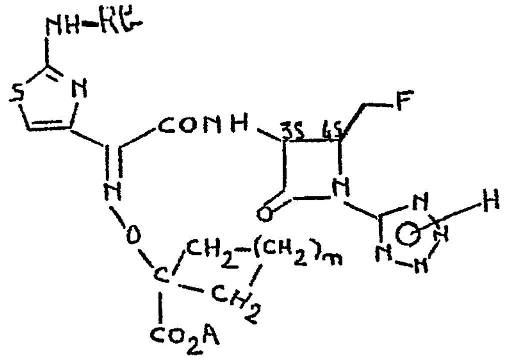

(IV)

in which n, Rb and A have the previous significance, which product is submitted, if necessary, to any of the following reactions, in any order:

a) cutting off by hydrolysis, hydrogenolysis or by the action of thiourea of the protector group or groups which Rb and A can represent;

b) salification of the carboxy radical;

c) salification by an acid of the amino radial.

2. Process according to claim 1, characterized in that at the start a product of formula III is used, in which Rb represents a trityl radical.

3. Process according to claim 1 or 2, characterized in that at the start a reactive derivative of the product of formula III is used which is a mixed carboxylic sulphonic anhydride.

4. Process according to any one of claims 1 to 3, characterized in that at the start compounds of formula II and III are used, chosen in such a way that the products of formula I are prepared, of which the names follow:

– (3S,4S) 1–[[[1-(2-amino-4-thiazolyl)-2-[[(4-fluoromethyl-2-oxo-1-tetrazolyl-3-azetidinyl)]amino]-2-oxoethylidene]amino]oxy]cyclopropane carboxylic acid and its salts,

– (3S,4S) 1-[[[1-(2-amino-4-thiazolyl)-2-[[(4-fluoromethyl-2-oxo-1-tetrazolyl-3-azetidinyl)]amino]-2-oxoethylidene]amino]oxy]cyclobutane carboxylic acid and its salts,

– (3S,4S) 1-[[[1-(2-amino-4-thiazolyl)-2-[[(4-fluoromethyl-2-oxo-1-tetrazolyl-3-azetidinyl)]amino]-2-oxoethylidene]amino]oxy]cyclopentane carboxylic acid and its salts.